# EUROPEAN PATENT SPECIFICATION

(11) **EP 4 633 613 B1**
(45) Date of publication and mention of the grant of the patent: **25.11.2026**
(21) Application number: 23832767.0
(22) Date of filing: 15.12.2023
(51) Int. Cl.: A61K 9/20, A61K 31/00, A61K 31/4545, A61K 31/47

(54) **PHARMACEUTICAL COMPOSITION COMPRISING RUPATADINE AND MONTELUKAST**
PHARMAZEUTISCHE ZUSAMMENSETZUNG MIT RUPATADIN UND MONTELUKAST
COMPOSITION PHARMACEUTIQUE COMPRENANT DE LA RUPATADINE ET DU MONTÉLUKAST

(30) Priority: 15.12.2022 IN 202211072708; 30.01.2023 EP 23382074
(43) Date of publication of application: 22.10.2025
(73) Proprietor: NOUCOR HEALTH S.A., Palau-solità i Plegamans 08184 (Barcelona) (ES); Intas Pharmaceuticals Ltd., Ahmedabad - 380054, Gujarat (IN)
(72) Inventor: STRUSI, Orazio Luca, 08206 Sabadell, Barcelona (ES); CODERCH, Montserrat, 08184 Palau-solità i Plegamans, Barcelona (ES); SEHGAL, Ashish, Ahmedabad, Gujarat 382 210 (IN); CHAUHAN, Manishkumar Jayantibhai, Ahmedabad, Gujarat 382 210 (IN); PANDYA, Kavan Asitkumar, Ahmedabad, Gujarat 382 210 (IN)
(74) Representative: ABG Intellectual Property Law, S.L.
(86) International application number: PCT/EP2023/086014
(87) International publication number: WO 2024/126772

(56) References cited:
- WO-A1-2015/069203
- WO-A1-2017/182641
- WO-A1-2017/182644

## Description

### FIELD OF THE INVENTION

The present invention relates to pharmaceutical compositions, in particular tablets, which contain the antihistamine rupatadine or salts thereof and the leukotriene D4 receptor antagonist montelukast or salts thereof to improve the therapy of allergic disorders.

### BACKGROUND OF THE INVENTION

The number of allergic disorders is increasing drastically worldwide. Intensive research activities of recent years have led to the recognition that allergic rhino conjunctivitis is an inflammatory process in the sense of a persistent inflammatory reaction. While histamine is still regarded as the most important mediator of the early phase and as the most important trigger of the symptoms such as reddening, sneezing, itching and hypersecretion (rhinorrhea and lacrimation), further mediators such as the leukotrienes are involved in the nasal obstruction, secretion and in the progression of the inflammation (e.g. attraction of the proinflammatory cells, promotion of cellular infiltration, etc.). Accordingly, the aims of the therapy have been shifted from symptomatic therapy to an additional anti-inflammatory therapy with influencing of the inflammation underlying the allergic disorders. Both histamine and leukotrienes (LTs) are released in the allergic early phase and late phase.

It has been proposed to combine antihistaminic agents with leukotriene D4 receptor antagonists to enhance the treatment of allergic rhinitis, vasomotor rhinitis, or allergic conjunctivitis (see CA 2 337 571 C).

WO 2015/069203 A1 discloses preparations comprising montelukast and rupatadine for use in the treatment of asthma accompanied by allergic rhinitis and allergic rhinitis. However, the document also discloses that incompatibility and stability problems frequently occur in the combinations of active substances in the same dosage form. In order to solve stability and incompatibility problems, the document proposes to combine, in a capsule, coated tablets comprising rupatadine fumarate with granules of montelukast sodium. Thus, the contact of montelukast and rupatadine active substances is minimized by being each active substance present in its own solid dosage form inside the capsule.

WO 2017/182641 A1 also discloses that combining montelukast and rupatadine in a tablet represents a great challenge because of compatibility, dissolution and stability problems. To overcome these problems, it is suggested to formulate the products in the form of a bilayer tablet formulation comprising montelukast sodium in a first layer and rupatadine fumarate in a second layer. Again, this approach minimizes the contact of montelukast and rupatadine by physical separation in different layers and, thus, in different pharmaceutical compositions. WO 2017/182641 A1 also shows that when rupatadine and montelukast are formulated together in one layer tablet form or in capsule form the dissolution and bioavailbilty of montelukast is significantly decreased.

WO 2017/182644 A1, which has the same filing date as WO 2017/182641 A1, discloses that, during studies conducted to combine montelukast and rupatadine, it was found that montelukast shows stability and dissolution problems when used together with rupatadine in a tablet formulation or in capsule form. According to said document, the direct combination of the two APIs in the same pharmaceutical composition, being it tablet or capsule form, causes an increase in impurity and a decrease in dissolution of montelukast. The alleged solution to the problem proposed in said application was to formulate the two substances together by spray granulation method only (for example in fluidized bed dryer), thus granulating rupatadine directly together with montelukast and excipients. However, this method requires the use of complex equipment and involves the need to spend a high amount of energy to obtain the product. Moreover, the document does not show that the known stability and dissolution problems have indeed been overcome and that no further incompatibility between the two active substances exists.

Thus, there is still the unsolved need to develop a formulation comprising rupatadine or its salts together with montelukast or its salts in a tablet form, which is simple *per se,* can be manufactured by simple methods, that does not involve complex equipment or the use of high amounts of energy and shows at the same time an excellent bioavailability of the combined substances montelukast and rupatadine. Additionally, there is a need to provide compositions of montelukast and rupatadine wherein montelukast remains stable against decomposition even in the presence rupatadine, since the montelukast reference product Singulair^{®} is known to have a rather short shelf life of only 2 years due to being moisture and light sensitive and must thus be protected therefrom and stored under controlled room temperature.

### SUMMARY OF THE INVENTION

The inventors have surprisingly found that when either rupatadine or a pharmaceutically acceptable salt thereof or montelukast or a pharmaceutically acceptable salt thereof are prepared in the form of granules which contain only one of two active substances, it is possible to combine both APIs even in a single layer tablet without having stability problems and at the same time showing an excellent bioavailability profile for both active ingredients. Contrary to the teachings of WO 2017/182641 A1, wherein it is shown that when rupatadine and montelukast are formulated together in one layer tablet form or in capsule form the dissolution and bioavailability of montelukast is significantly decreased, in the present invention it has been surprisingly found that it is possible to combine both rupatadine and montelukast even in a single layer tablet and, despite the worsened dissolution profile obtained for each of the actives in the single layer tablet when compared to the combination of individual tablets of montelukast (Singulair^{®}) and rupatadine (Rupafin^{®}), the formulation of the invention is bioequivalent to the coadministration of individual montelukast (Singulair^{®}) and rupatadine (Rupafin^{®}) commercial formulations. Also, montelukast has an impurity profile under storage experiments that shows that it remains at least as stable as its reference product Singulair^{®}, if not even better.

Thus, in the first aspect, the invention relates to a pharmaceutical composition in the form of a single layer tablet comprising rupatadine or a pharmaceutically acceptable salt thereof and montelukast or a pharmaceutically acceptable salt thereof, wherein:
a) at least one of i) rupatadine or a pharmaceutically acceptable salt thereof and ii) montelukast or a pharmaceutically acceptable salt thereof is present in the form of granules together with at least one pharmaceutically acceptable excipient and
b) said granules do not contain at the same time i) rupatadine or a pharmaceutically acceptable salt thereof and ii) montelukast or a pharmaceutically acceptable salt thereof.

In a second aspect, the invention relates to a pharmaceutical composition as defined in the first aspect, for use in medicine.

In a third aspect, the invention relates to a pharmaceutical composition as defined the first aspect, for use in the treatment and/or prevention of asthma accompanied by allergic rhinitis and allergic rhinitis.

The term "prevention", as used herein, refers to the administration of the composition of the invention in an initial or early stage of a disease, or to also prevent its onset.

The term "treatment" is used to designate the administration of the composition of the invention to control disorder progression before or after the clinical signs had appeared. By control of the disorder progression it is meant to designate beneficial or desired clinical results including, but not limited to, reduction of symptoms, reduction of the length of the disorder, stabilization pathological state (specifically avoidance of further deterioration), delay in the disorder's progression, improvement of the pathological state and remission (both partial and total). In a particular embodiment of the invention the composition of the invention is used to control the disorder progression once at least one of the disorder's clinical signs has appeared.

The term "medicament", as used herein, refers to a composition of the invention..

The term "subject", as used herein, refers to any animal or human that is suffering from one of the diseases disclosed above. Preferably, the subject is a mammal. The term "mammal", as used herein, refers to any mammalian species, including but not being limited to domestic and farm animals (cows, horses, pigs, sheep, goats, dogs, cats or rodents), primates, and humans. Preferably, the mammal is a human being.

### DESCRIPTION OF THE FIGURES

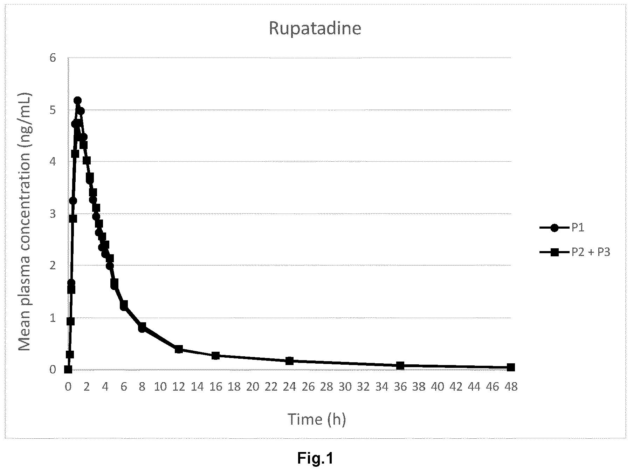

Figure 1 shows the mean plasma concentration (ng/ml) of rupatadine in products P1 and P2 (co-administered with P3) obtained in the clinical trial described in the example section.

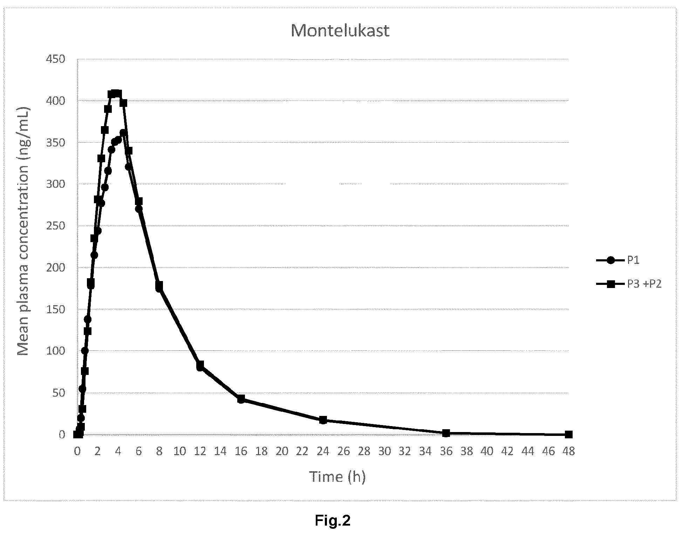

Figure 2 shows the mean plasma concentration (ng/ml) of montelukast in products P1 and P3 (co-administered with P2) obtained in the clinical trial described in the example section.
Figure 3 shows the dissolution profile of montelukast in the tablet of example 1 (black) and in the combination of Rupafin^{®}+Singulair^{®} (grey).
Figure 4 shows the dissolution profile of rupatadine in the tablet of example 1 (black) and in the combination of Rupafin^{®}+Singulair^{®} (grey).

### DETAILED DESCRIPTION

In one aspect the invention relates to a pharmaceutical composition in the form of a single layer tablet comprising rupatadine or a pharmaceutically acceptable salt thereof and montelukast or a pharmaceutically acceptable salt thereof, wherein:
a) at least one of i) rupatadine or a pharmaceutically acceptable salt thereof and ii) montelukast or a pharmaceutically acceptable salt thereof is present in the form of granules together with at least one pharmaceutically acceptable excipient and
b) said granules do not contain at the same time i) rupatadine or a pharmaceutically acceptable salt thereof and ii) montelukast or a pharmaceutically acceptable salt thereof.

In the context of the present invention the term "granules" is used to designate a plurality of solid particles that preferably have a bulk density of at least 0.3 g/ml, preferably at least 0.35 g/ml and most preferably at least 0.37 g/ml measured using a graduated cylinder.

In one embodiment, the granules of the present invention have a size such that at least 90% of said particles is retained by a sieve having circular holes with a diameter of 0.1 mm and that not more than 20% of said particles is retained by a sieve having circular holes with a diameter of 1.0 mm.

In the context of the present invention the term "rupatadine" means rupatadine free base or any pharmaceutically acceptable salt thereof. Preferably, it means a pharmaceutically acceptable salt of rupatadine being selected from the group consisting of hydrochloride salt, hydrobromide salt, hydroiodide salt, nitrate salt, perchlorate salt, sulfate salt, phosphate salt, methanesulfonate salt, trifluoromethanesulfonate salt, ethanesulfonate salt, benzenesulfonate salt, p-toluenesulfonate salt, fumarate salt, oxalate salt, maleate salt, citrate salt, succinate salt. More preferably, it means rupatadine fumarate salt.

In the context of the present invention the term "montelukast" means montelukast free acid or any pharmaceutically acceptable salt thereof. Preferably, it means a pharmaceutically acceptable salt of montelukast being selected from the group consisting of lithium salt, sodium salt, potassium salt, ammonium salt, calcium salt, magnesium salt, arginine salt, betaine salt, caffeine salt, choline salt, N,N'-dibenzylethylenediamine salt, diethylamine salt, 2-diethylaminoethanol salt, 2-dimethylaminoethanol salt, ethanolamine salt, ethylenediamine salt, N-ethylmorpholine salt, N-ethylpiperidine salt, glucamine salt, glucosamine salt, histidine salt, hydrabamine salt, isopropylamine salt, lysine salt, methylglucamine salt, morpholine salt, piperazine salt, piperidine salt, theobromine salt, triethylamine salt, trimethylamine salt, tripropylamine salt, tromethamine salt, dicyclohexylamine salt. More preferably, it means montelukast sodium salt.

In one embodiment, the single layer tablet, comprises rupatadine or a pharmaceutically acceptable salt thereof, preferably rupatadine fumarate, in the form of granules wherein the granules do not comprise montelukast or a pharmaceutically acceptable salt thereof. In a particular embodiment montelukast or a pharmaceutically acceptable salt thereof is added in extra-granular powder form, optionally together with additional excipients to the granules comprising rupatadine or a pharmaceutically acceptable salt thereof.

In another embodiment, the single layer tablet comprises montelukast or a pharmaceutically acceptable salt thereof, preferably montelukast sodium, in the form of granules wherein the granules do not comprise rupatadine or a pharmaceutically acceptable salt thereof.

In one embodiment, the granules comprising either rupatadine or a pharmaceutically acceptable salt thereof or montelukast or a pharmaceutically acceptable salt thereof are prepared by a granulation method different from spray granulation.

In one embodiment, the granules comprising either rupatadine or a pharmaceutically acceptable salt thereof or montelukast or a pharmaceutically acceptable salt thereof are prepared by a granulation method selected from the group consisting of dry granulation, wet granulation by low or high shear mixer and hot melt granulation. Most preferably, the granules are prepared by wet granulation in a high-shear mixer which is capable of providing one or more of the following advantages:
1. Short processing time
2. Reduced binder solution quantity
3. Ability to process highly cohesive materials
4. Greater densification and reduced granule friability
5. Reproducibility of uniform granule size distribution
6. Dust reduction
7. Predictable end-point determination

In one embodiment, the pharmaceutical composition comprises one or more excipients selected from the group consisting of binders, diluents, disintegrants, lubricants, glidants, antioxidants, colorants and flavors.

Disintegrants may aid dispersion of the tablet in the gastrointestinal tract, releasing the active ingredient and increasing the surface area for dissolution. According to one embodiment, disintegrants may be selected from the group comprising alginates such as calcium and/or sodium alginate, calcium carboxymethylcellulose, calcium cellulose glycolate, calcium silicate, carboxy methyl cellulose calcium, starch, croscarmellose sodium, crospovidone, sodium docusate, hydroxypropyl methyl cellulose, hydroxypropyl starch, magnesium aluminum silicate, methylcellulose, polyvinylpyrrolidone, powdered cellulose, sodium carboxy methyl cellulose, sodium starch glycolate, and mixtures thereof. In an embodiment, the disintegrant preferably is sodium croscarmellose
Binders help binding the tablet ingredients together giving form and mechanical strength. According to one embodiment, binders may be selected from the group consisting of acacia mucilage, agar, alginates (sodium), carboxymethylcellulose (sodium or calcium), carbomer, carrageenan, chitosan, copovidone, starch (corn), ethylcellulose, gelatin, hydroxyethyl cellulose, hydroxyethylmethyl cellulose, hydroxypropyl cellulose, hydroxypropyl methyl cellulose, hydroxypropyl starch, magnesium aluminum silicate, methyl cellulose, natural gums (agar, guam, tragachanti), pectin, polyethylene glycols, polyvinylpyrrolidone, pregelatinized starch, and mixtures thereof. In an embodiment, the binders preferably are pregelatinized starch and hydroxypropyl cellulose.

Diluents (also known as fillers) provide bulk and enable accurate dosing of active ingredients. According to one embodiment, binders may be selected from the group consisting of alpha-lactalbumin, anhydrous lactose, calcium carbonate, calcium lactate, calcium phosphate (dibasic, anhydrous and dibasic, dehydrate), calcium sulfate, cellulose acetate, compressible sugar, dextrates, dextrin, dextrose, erythritol, ethyl acrylate and methyl methacrylate copolymer, ethylcellulose, fructose, hydroxypropyl pea starch, isomalt, kaolin, lactitol, lactose monohydrate, magnesium carbonate, magnesium oxide, maize starch, maltodextrin, maltose, mannitol, methacrylic acid and ethyl acrylate copolymer, methacrylic acid and methyl methacrylate copolymer, microcrystalline cellulose, polydextrose, powdered cellulose, pregelatinized hydroxypropyl potato starch, pregelatinized modified starch, starch (corn, potato, maize, pea, tapioca, wheat), pullulan, silicified microcrystalline cellulose, simethicone, sodium bicarbonate, sodium carbonate, sodium chloride, sorbitol, spray-dried lactose, hydrogenated starch hydrolysate, sucrose, trehalose, tribasic calcium phosphate, xylitol and mixtures thereof. In an embodiment, the diluents preferably are lactose monohydrate and microcrystalline cellulose.

Lubricants (also known as glidants) improve the flow of powders during tablet manufacturing by reducing friction and adhesion between particles. According to one embodiment, lubricants may be selected from the group consisting of behenoyl polyoxylglycerides, calcium silicate, calcium stearate, cellulose, powdered colloidal silicon dioxide, fumaric acid, glyceryl behenate, glyceryl dibehenate, glyceryl monostearate, glyceryl palmitostearate, glyceryl tristearate, hydrogenated natural oils, hydrophobic colloidal silica, lauric acid, magnesium lauryl sulphate, magnesium lauryl sulphate, magnesium oxide, magnesium silicate, magnesium stearate, myristic acid, palmitic acid, paraffin, poloxamer, polyethylene glycol, polyethylene glycol, polyethylene glycol, polyoxyl 10 oleyl ether, polyoxyl 15 hydroxystearate, polyoxyl 20 cetostearyl ether, polyoxyl 40 stearate, polysorbates (20, 40, 60, 80), potassium benzoate, silica, sodium benzoate, sodium lauryl sulphate, sodium stearate, sodium stearyl fumarate, sorbitan monolaurate, sorbitan monooleate, sorbitan monopalmitate, sorbitan monostearate, sorbitan sesquioleate, sorbitan trioleate, stearic acid, talc, tribasic calcium phosphate, zinc stearate and mixtures thereof. In an embodiment, the lubricant preferably is magnesium stearate.

In one embodiment the pharmaceutical composition of the invention has a total amount of diluents comprised between 20 wt% and 90 wt% w/w, preferably between 35 wt% and 90 wt%, more preferably between 50 wt% and 90 wt% and more preferably between 70 wt% and 90 wt%, a total amount of binders comprised between 2 and 14 wt%, preferably between 2 wt% and 11 wt%, more preferably between 3 and 9% and more preferably between 4 wt% and 7 wt%, a total amount of disintegrants comprised between 1 and 15 wt %, preferably between 1 wt% and 13 wt%, more preferably between 1 and 11% and more preferably between 1 wt% and 8 wt% and a total amount of lubricants comprised between 0.2 and 2.0 wt%, preferably between 0.3 wt% and 1.7 wt%, more preferably between 0.5 and 1.5% and more preferably between 0.7 wt% and 1.3 wt% all percentages being expressed relative to the total weight of the pharmaceutical composition.

In one embodiment the pharmaceutical composition of the invention comprises as pharmaceutical excipients, pregelatinized starch, microcrystalline cellulose, lactose monohydrate, hydroxypropyl cellulose, sodium croscarmellose and magnesium stearate.

In one embodiment the pharmaceutical composition of the invention has a total amount of pregelatinized starch comprised between 1 wt% and 10 wt% w/w, preferably between 1.5 wt% and 8 wt%, more preferably between 2 wt% and 6 wt% and more preferably between 2.5 wt% and 4 wt%, a total amount of microcrystalline cellulose comprised between 10 wt% and 60 wt% w/w, preferably between 18 wt% and 52 wt%, more preferably between 25 wt% and 45 wt% and more preferably between 32 wt% and 38 wt%, a total amount of lactose monohydrate comprised between 15 wt% and 75 wt% w/w, preferably between 23 wt% and 68 wt%, more preferably between 30 wt% and 60 wt% and more preferably between 35 wt% and 55 wt%, a total amount of hydroxypropyl cellulose comprised between 0.5 wt% and 8 wt% w/w, preferably between 0.8 wt% and 8 wt%, more preferably between 1 wt% and 6 wt% and more preferably between 1.5 wt% and 3 wt%, a total amount of sodium croscarmellose comprised between 0.5 wt% and 8 wt% w/w, preferably between 0.8 wt% and 8 wt%, more preferably between 1 wt% and 6 wt% and more preferably between 1.5 wt% and 3 wt% and a total amount of magnesium stearate comprised between 0.1 wt% and 2 wt% w/w, preferably between 0.3 wt% and 1.6 wt%, more preferably between 0.5 wt% and 1.4 wt% and more preferably between 0.8 wt% and 1.2 wt%,

In one embodiment, each tablet of the pharmaceutical composition has an amount of rupatadine or a pharmaceutically acceptable salt thereof per pharmaceutical composition in the tablet of from 0.5 to 22 mg, preferably from 1.5 to 20 mg, more preferably from 2 to 17 mg, more preferably from 4 to 15 mg, and even more preferably from 7 to 12 mg, expressed as the weight of rupatadine free base. In a more preferred embodiment, rupatadine or a pharmaceutically acceptable salt thereof is present in an amount of about 10 mg expressed as rupatadine free base.

In one embodiment, each tablet of the pharmaceutical composition has an amount of montelukast or a pharmaceutically acceptable salt thereof per pharmaceutical composition in the tablet of from 1 to 22 mg, preferably from 1.5 to 20 mg, more preferably from 2 to 17 mg, more preferably from 4 to 15 mg, and even more preferably from 7 to 12 mg, expressed as weight of montelukast free acid. In a more preferred embodiment, montelukast or a pharmaceutically acceptable salt thereof is present in an amount of about 10 mg expressed as montelukast free acid.

In one embodiment, in the pharmaceutical composition according to the invention the weight-to-weight ratio of rupatadine, calculated based on the weight of rupatadine free base, to the disintegrant is of from 5:1 to 1:5, preferably of from 3.5:1 to 1:3.5, and more preferably of from 2.5:1 to 1:2.5.

In one embodiment, in the pharmaceutical composition according to the invention the weight-to-weight ratio of montelukast, calculated based on the weight of montelukast free base, to the disintegrant is of from 5:1 to 1:5, preferably of from 3.5:1 to 1:3.5, and more preferably of from 2.5:1 to 1:2.5.

In a preferred embodiment the compositions of the invention comprise rupatadine or a pharmaceutically acceptable salt thereof in the form of granules.

The pharmaceutical composition of the invention shows an excellent bioavailability of both APIs, i.e. of both rupatadine and montelukast, expressed by the in vivo results obtained for the maximum plasma concentration (Cₘₐₓ) and the area under the plasma concentration vs time curve (AUC₀₋ₜ), explained in the following.

In an embodiment, the pharmaceutical composition of the invention when administered orally exhibits a maximum plasma concentration (Cₘₐₓ) of Rupatadine from 5.00 ng/mL to 6.50 ng/mL, preferably from 5.20 ng/mL to 6.40 ng/mL and more preferably from 5.30 ng/mL to 6.30 ng/mL and a maximum plasma concentration (Cₘₐₓ) of Montelukast from 380 ng/mL to 530 ng/mL, preferably from 395 ng/mL to 520 ng/mL and more preferably from 410 ng/mL to 510 ng/mL.

In an embodiment, the pharmaceutical composition of the invention when administered orally exhibits an area under the plasma concentration vs. time curve (AUC₀₋ₜ) of Rupatadine from 0 to 48 hours from 24.5 ng·h/mL to 28.5 ng·h/mL, more preferably from 25.0 ng·h/mL to 28.0 ng·h/mL and more preferably from 25.5 ng·h/mL to 27.5 ng·h/mL and an area under the plasma concentration vs. time curve (AUC₀₋ₜ) of Montelukast from 0 to 48 hours from 2850.0 ng·h/mL to 3450.0 ng·h/mL, more preferably from 2900.0 ng·h/mL to 3400.0 ng·h/mL and more preferably from 2950.0 ng·h/mL to 3350.0 ng·h/mL.

The data presented in Example 2 below show that the pharmaceutical compositions of the present invention are bioequivalent (both with respect of rupatadine and with respect of montelukast) with a co-administration of separate oral dosage forms of Rupatadine and Montelukast.

In an embodiment, the pharmaceutical composition of the invention comprises rupatadine in the form of rupatadine fumarate.

In an embodiment, the pharmaceutical composition of the invention comprises montelukast in the form of montelukast sodium.

The compositions of the present invention have in common that at least one of the active ingredients (rupatadine or its salts / montelukast or its salts) is prepared in the form of granules, which do not contain at the same time both active ingredients. This can be achieved in various ways:
a) only rupatadine or its salts is prepared in the form of granules comprising also at least one excipient but not comprising montelukast or its salts;
b) only montelukast or its salts is prepared in the form of granules comprising also at least one excipient but not comprising rupatadine or its salts;
c) rupatadine or its salts is prepared in the form of granules comprising also at least one excipient but not comprising montelukast or its salts and montelukast or its salts is prepared in the form of granules comprising also at least one excipient but not comprising rupatadine or its salts.

Hence, the compositions so obtained have the advantage that any drug-drug interaction is minimized by separating them physically. This allows for obtaining compositions showing excellent drug release of both active ingredients with an excellent bioavailability that shows bioequivalence to each active ingredient administered individually. Further, the compositions of the present invention do not affect the stability of montelukast, which is known to be a critical issue, maintaining at least its stability performance at the same level as the reference drug product Singulair^{®}.

The preparation of the granules comprising one of the active ingredients may be carried out by any granulation method different from spray granulation. It is preferred to use a granulation method selected from the group consisting of dry granulation, wet granulation by low or high shear mixer and hot melt granulation. Most preferably, the granules are prepared by wet granulation in a high-shear mixer. This is a very straightforward and simple manufacturing method that does not require any special conditions.

In an embodiment of the present invention, the granules comprising the active material also comprise one or more of binders, diluents and disintegrants.

When only one of rupatadine (or its salts) and montelukast (or its salts) is used in the form of granules to prepare the composition ready to be tableted, i.e. is used intragranular, the ingredient which is not present in granular form is said to be extragranular and thus mixed with the granules and with any other necessary additional excipient, such as binders, diluents, disintegrants, lubricants or any other pharmaceutically acceptable additive.

When both rupatadine (or its salts) and montelukast (or its salts) are used in the form of granules to prepare the composition ready to be tableted, i.e. are used intragranular, both types of granules are mixed with any other necessary additional excipient, such as binders, diluents, disintegrants, lubricants or any other pharmaceutically acceptable additive.

In a preferred embodiment of the present invention, rupatadine or a salt thereof is mixed with a binder and one or more diluents and the mixture is granulated with water or with an aqueous solution with a binder dissolved in it. In a preferred embodiment, the granulation is carried out in a high-shear mixer.

In a preferred embodiment of the present invention, the granules comprising rupatadine or its salts are mixed extragranular with montelukast or its salts in the form of dry powder and with one or more of binders, diluents, disintegrants and lubricants or any other pharmaceutically additive to obtain a pharmaceutical composition. The thus obtained pharmaceutical composition may be used as such, may be filled into capsules or compressed into tablets. Preferably, the pharmaceutical composition is compressed into tablets by using, for example, a rotary tablet press.

After tableting, the tablets may be used directly without any coating or they may optionally be coated with a coating solution, such as an aqueous solution comprising polyvinyl alcohol, hydroxypropylmethylcellulose, methylcellulose and colorants or pigments. Preferably, the tablets remain uncoated.

It has been reported that rupatadine and montelukast can be used for the treatment of asthma accompanied by allergic rhinitis and allergic rhinitis (WO 2015/069203 A1).

Thus, in a second aspect, the invention relates to a pharmaceutical composition as defined in the first aspect, for use in medicine.

In a third aspect, the invention relates to a pharmaceutical composition as defined the first aspect, for use in the treatment and/or prevention of asthma accompanied by allergic rhinitis and allergic rhinitis.

The following examples represent specific embodiments of the present invention. They do not intend to limit in any way the scope of the invention defined in the present description.

### EXAMPLES

### Example 1

300 mg tablets were prepared with the composition showed in Table 1 following the process described below:

**Table 1**

| # | Ingredient | % | Weight/tablet (mg) |
|---|---|---|---|
| 1 | Rupatadine fumarate (as rupatadine free base) | 4.27 | 12.8 (10.0) |
| 2 | Pregelatinized starch | 3.33 | 10.0 |
| 3 | Microcrystalline cellulose | 5.0 | 15.0 |
| 4 | Lactose monohydrate | 20.4 | 61.2 |
| 5 | Montelukast sodium (as Montelukast free acid) | 3.47 | 10.4 (10.0) |
| 6 | Lactose monohydrate | 29.33 | 88.0 |
| 7 | Microcrystalline cellulose | 29.29 | 87.87 |
| 8 | Hydroxypropyl cellulose | 1.91 | 5.73 |
| 9 | Sodium croscarmellose | 2.0 | 6.0 |
| 10 | Magnesium stearate | 1.0 | 3.0 |
| | TOTAL | 100.0 | 300.0 |

### Step 1: Preparation of rupatadine fumarate granulate

128 g of rupatadine fumarate, 100 g of pregelatinized starch, 150 g of microcrystalline cellulose and 612 g of lactose monohydrate were blended to prepare a homogeneous mixture. The mixture was then granulated with 216.81 g of water in a high-shear mixer (Mixer-granulator Lleal TRI-CHOP MGR-5/1). The resulting wet granulate was dried in an air-forced oven at a temperature of 45°C.

### Step 2: Preparation of intermediate mixture

768.86 g of microcrystalline cellulose, 770.0 g of lactose monohydrate, 91 g of sodium montelukast and 50.14 g of hydroxypropyl cellulose were blended to prepare a homogeneous mixture.

### Step 3: Preparation of product for compression

52.5 g of sodium croscarmellose were added to 866.25 g of the rupatadine fumarate granules prepared in step 1 followed by 1680.0 g of intermediate mixture prepared in step 2 and the mixture was blended to obtain a homogeneous mixture. Finally, 26.25 mg of magnesium stearate were added to the previous mixture and the mixture was blended to obtain a homogeneous mixture.

### Step 4: Preparation of tablets

The final mixture of step 3 was compressed to a 300.0 mg tablets in a rotary tablet press equipped with round punches having a diameter of 9 mm at a pressure of 4-5 KN.

Example 2: An open label, balanced, randomized, four-period, single oral dose, crossover, study to evaluate the relative bioavailability and to establish bioequivalence of test formulation (Rupatadine 10 mg + Montelukast 10 mg) versus Rupatadine 10 mg and Montelukast 10 mg co-administered as separate oral dosage forms in normal, healthy, adult, human subjects under fasting condition

A Phase-I Study was carried out to assess (among other variables) the bioequivalence of the test formulation (Rupatadine 10 mg + Montelukast 10 mg) versus Rupatadine 10 mg and Montelukast 10 mg co-administered as separate oral dosage forms in normal, healthy, adult, human subjects under fasting condition.. The following three formulations were used in the study:

| | |
|---|---|
| P1- | Rupatadine 10 mg / Montelukast 10 mg tablets (product of example 1) |
| P2- | Tamalis^{®} (Rupatadine) 10 mg tablets |
| P3- | Singulair^{®} (Montelukast) 10 mg tablets |

Sixty (60) volunteers were considered eligible as per protocol and enrolled on check-in day. From these only 53 subjects completed the 4 dosing periods and were considered for the Bioequivalence statistical analysis.

Products P1 to P3 where used in the following treatments:
Treatment-T: 1 tablet per administration of product P1 (Rupatadine + Montelukast)
Treatment-R1: 1 tablet per administration of product P3 (Montelukast)
Treatment-R2: 1 tablet per administration of product P2 (Rupatadine)
Treatment-R: 1 tablet per administration of product P2 (Rupatadine) + 1 tablet per administration of product P3 (Montelukast)

The treatments were carried out according to the following sequences:

**Table 2 Treatment sequence**

| Period I | Period II | Period III | Period IV |
|---|---|---|---|
| Treatment-R1 | Treatment-R2 | Treatment-R | Treatment-T |
| Treatment-T | Treatment-R1 | Treatment-R2 | Treatment-R |
| Treatment-R | Treatment-T | Treatment-R1 | Treatment-R2 |
| Treatment-R2 | Treatment-R | Treatment-T | Treatment-R1 |

After an overnight fast of at least 10 hours, a single oral dose of either one tablet of test product (Rupatadine 10 mg + Montelukast 10 mg) or one tablet of P3 (10 mg of montelukast sodium) or one tablet of P2 (10 mg of Rupatadine) or co-administration of P2 and P3 was administered with 240 mL of drinking water at ambient temperature to the subjects in sitting posture. The administration of the products was as per the randomisation schedule and under open-label conditions.

The tablet was swallowed whole without chewing or crushing. The time of administration of the investigational medicinal product was the time at which the subject completed drinking 240 mL of water and that was captured in the respective source data forms. The entire activity was done under sodium vapour lamp covered with red gelatin paper.

This activity was followed by a mouth check to assess the compliance to dosing.

Subjects were in sitting position at chair side for the first 4 hours post-dose in each period unless medically necessary due to adverse event or procedurally required or natural exigency, in those cases it was not considered as protocol deviation.

Thereafter, the subjects were allowed to engage only in normal activities while avoiding strenuous physical activity. They refrained from drinking water from 1 hour prior to dosing till 2 hours after dosing in each period, except for the water given during product administration.

A total of twenty-five (25) blood samples, each of 5.5 mL were collected from each subject in each period, when the Montelukast and Rupatadine were given treatments T, R, R1 and R2. The venous blood samples were withdrawn at pre-dose (0.000 hour) and at 0.167, 0.250, 0.333, 0.500, 0.750, 1.000, 1.333, 1.667, 2.000, 2.333, 2.667, 3.000, 3.333, 3.667, 4.000, 4.500, 5.000, 6.000, 8.000, 12.000, 16.000, 24.000, 36.000 and 48.000 hours following administration of the products in each period.

The pharmacokinetic parameters (Cₘₐₓ and AUC₀₋ₜ) were calculated from the plasma concentration vs. time profile by non-compartmental model using Phoenix^{®} WinNonlin^{®} Version 8.1 (Certara L.P.) for Montelukast and Rupatadine. Statistical comparison of the pharmacokinetic parameters of the two formulations was carried out using PROC MIXED of SAS^{®} Version 9.4 (SAS Institute Inc., USA) to assess the bioequivalence between test and reference formulations.

The maximum measured plasma concentration (Cₘₐₓ) was calculated from the plasma concentration vs. time profile of the individual subjects. The units of Cmax are ng/mL. The area under the plasma concentration vs. time curve (AUC₀₋ₜ) was calculated by linear trapezoidal rule from measured data points from the time zero to the time of the last observed concentration. The units of AUC₀₋ₜ are ng·h/mL.

### Statistical methods:

Descriptive statistics are calculated and reported for Cₘₐₓ and AUC₀₋ₜ of Montelukast and Rupatadine. ANOVA, power and ratio analysis for In-transformed pharmacokinetic parameters Cₘₐₓ and AUC₀₋ₜ are calculated and reported for Montelukast and Rupatadine.

90% confidence intervals for the ratio of the geometric least squares means between drug formulations are calculated for In-transformed pharmacokinetic parameters Cₘₐₓ and AUC₀₋ₜ of Montelukast and Rupatadine.

To determine bioequivalence between the products P1 and the coadministration of products P2 and P3 with respect to Rupatadine the following is calculated:
- ratio of In-transformed Cₘₐₓ of Rupatadine for Treatment T to In-transformed Cₘₐₓ of Rupatadine for Treatment-R and 90% Confidence Interval for said ratio
- ratio of In-transformed AUC₀₋ₜ of Rupatadine for Treatment-T to In-transformed AUC₀₋ₜ of Rupatadine for Treatment-R and 90% Confidence Interval for said ratio

Two products are considered to be bioequivalent with respect of Rupatadine if the calculated 90% Confidence Intervals of the corresponding ratios fall within the range of 80.00 to 125.00%.

Similarly, to determine bioequivalence between the products P1 and the coadministration of products P2 and P3 with respect to Montelukast the following is calculated:
- ratio of In-transformed Cₘₐₓ of Montelukast for Treatment T to In-transformed Cₘₐₓ of Montelukast for Treatment-R and 90% Confidence Interval for said ratio
- ratio of In-transformed AUC₀₋ₜ of Montelukast for Treatment-T to In-transformed AUC₀₋ₜ of Montelukast for Treatment-R and 90% Confidence Interval for said ratio Two products are considered to be bioequivalent with respect of Montelukast if the calculated 90% Confidence Intervals of the corresponding ratios fall within the range of 80.00 to 125.00%.

**Table 3: Pharmacokinetic parameters of rupatadine in treatments T (product P1) and R (products P2 and P3)**

| **PK parameter** | **Treatment T (product P1 - Example 1)** | **Treatment R (products P2 and P3)** |
|---|---|---|
| Cₘₐₓ (ng/mL) | 5.856 ± 3.2636 | 5.617 ± 2.7455 |
| AUC₀₋₁ (ng·h/mL) | 26.345 ± 16.6220 | 26.358 ± 15.2279 |

**Table 4: Relative bioavailability results for Rupatadine**

| **PK parameter** | **Treatment T (product P1 - Example 1)** | **Treatment R (products P2 and P3)** | **T/R ratio** | **90% Confidence Interval** |
|---|---|---|---|---|
| In Cₘₐₓ | 5.006 | 4.991 | 100.3 | 93.16 - 108.01 |
| In AUC₀₋ₜ | 21.956 | 22.458 | 97.8 | 91.62 - 104.32 |

Since the calculated 90% Confidence Intervals of the corresponding ratios fall within the range of 80.00 to 125.00%, product P1 is bioequivalent (with respect of Rupatadine) with the coadministration of products P2 and P3.

**Table 5: Pharmacokinetic parameters of montelukast in treatments T (product P1) and R (products P2 and P3)**

| **PK parameter** | **Treatment T (product P1 - Example 1)** | **Treatment R (products P2 and P3)** |
|---|---|---|
| Cₘₐₓ (ng/mL) | 472.544 ± 134.4691 | 442.452 ± 165.9085 |
| AUC₀₋ₜ(ng·h/mL) | 3232.323 ± 965.5072 | 3009.230 ± 1200.7243 |

**Table 6: Relative bioavailability results for Montelukast**

| **PK parameter** | **Treatment T (product P1 - Example 1)** | **Treatment R (products P2 and P3)** | **T/R ratio** | **90% Confidence Interval** |
|---|---|---|---|---|
| In Cₘₐₓ | 451.466 | 409.602 | 110.2 | 99.08 - 122.62 |
| In AUC₀₋ₜ | 3085.481 | 2764.444 | 111.6 | 102.06 - 122.06 |

Since the calculated 90% Confidence Intervals of the corresponding ratios fall within the range of 80.00 to 125.00%, product P1 is bioequivalent (with respect of Montelukast) with the coadministration of products P2 and P3.

The product of example 1 (P1) when compared with the co-administration of the reference products Tamalis^{®} 10 mg tablets (P2) + Singulair^{®} 10 mg tablets (P3) meets the bioequivalence criteria with respect to Cₘₐₓ and AUC₀₋ₜ for Montelukast and Rupatadine under fasting condition as the 90% confidence interval (CI) falls within the acceptance range of 80.00-125.00% for In-transformed pharmacokinetic parameters.

A stability test was also performed to compare the stability of the composition of Example 1 versus Singulair^{®} because Montelukast is a known product prone to degradation. The results are represented in Table 7 as quantity of increased impurity vs. release.

**Table 7: Stability results composition of Example 1 vs Singulair^{®}**

| Temperature = 25 °C / Relative humidity = 60 % | | | | |
|---|---|---|---|---|
| | t = 3 months | | t = 6 months | |
| | Singulair | Example 1 | Singulair | Example 1 |
| Impurity C | + 0.06% | +0.03% | +0.06% | +0.03% |
| Impurity F | +0.03% | <0.001% | +0.03% | <0.001% |

| Temperature = 30 °C / Relative humidity = 75 % | | | | |
|---|---|---|---|---|
| Impurity C | + 0.04% | +0.05% | +0.45% | +0.07% |
| Impurity F | +0.01% | <0.001% | <0.001% | <0.001% |

| Temperature = 40 °C / Relative humidity = 75 % | | | | |
|---|---|---|---|---|
| Impurity C | +0.24% | +0.14% | | |
| Impurity F | +0.05% | <0.001% | | |

The level of impurities were measured by UHPLC using a Acquity UPLC BEH C18 1.7 µm (100 x 2.1 mm) column and the following mobile phase:

| t (minutes) | % Acetonitrile | % Ammonium acetate (10 mM , pH =5) |
|---|---|---|
| 0.0 | 10 | 90 |
| 0.5 | 10 | 90 |
| 5.0 | 46 | 54 |
| 19.0 | 60 | 40 |
| 23.0 | 90 | 10 |
| 25.0 | 10 | 90 |
| 26.0 | 10 | 90 |

The detection wavelength was fixed at 238 nm.

Impurity C appeared at 9.5 minutes, Montelukast at 18.3 min and Impurity F at 19.0 min.

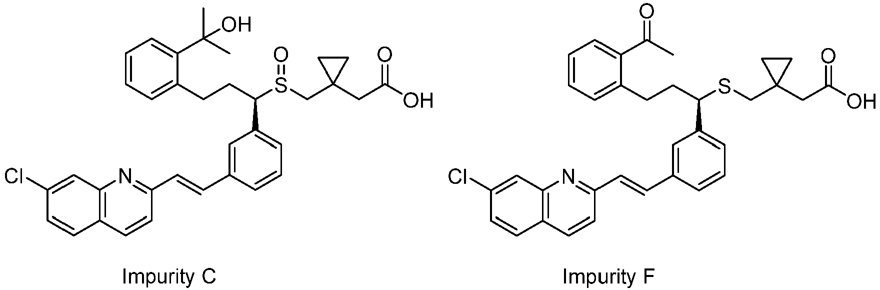

As can be seen from Table 7, both impurities show a better behaviour in Example 1 compared to the reference Montelukast product.

Example 3. Dissolution profile of the tablet of example 1 and the combination of Rupafin^{®}+Singulair^{®}.

The dissolution profile of montelukast and rupatadine in the tablets obtained in Example 1 was compared with that of the combination of montelukast (Singulair^{®}) and rupatadine (Rupafin^{®}).

The composition of each Singulair^{®} tablet is as follows:
- 10.00 mg montelukast (as the sodium salt)
- 5.73 mg hydroxypropylcellulose
- 89.30 mg microcrystalline cellulose
- 89.30 mg lactose monohydrate
- 6.00 mg sodium croscarmellose
- 1.00 mg magnesium stearate
- 1.73 mg hydroxypropylmethylcellulose
- 1.50 mg titanium dioxide
- 0.004 mg red iron oxide
- 0.036 mg yellow iron oxide
- 0.006 mg carnauba wax

The composition of each Rupafin^{®} tablet is as follows:
- 10 mg of rupatadine (as fumarate)
- pregelatinised maize starch
- microcrystalline cellulose
- red iron oxide (E-172)
- yellow iron oxide (E-172)
- lactose monohydrate, 57.57 mg
- magnesium stearate

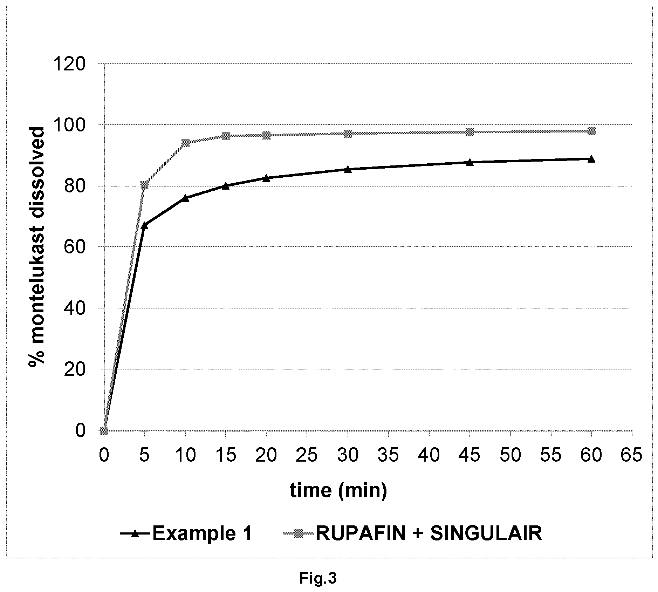

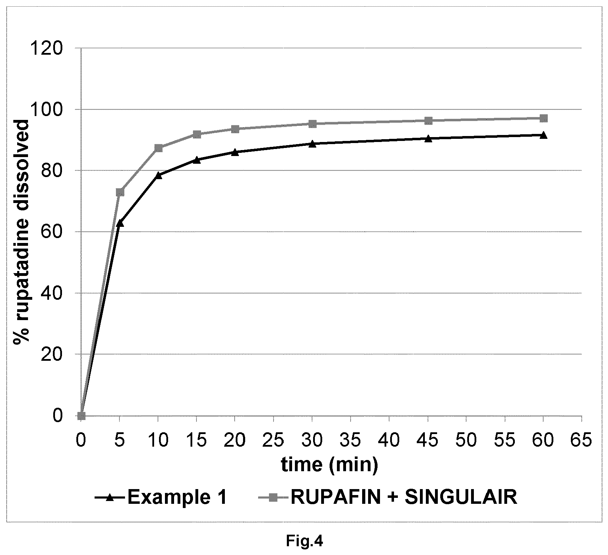

The dissolution tests were carried out according to the paddle method (European Pharmacopeia, 2.9.3) at 50 rpm and 37 °C in a solution of 0.5% sodium dodecyl sulfate (SDS) in water. The results are shown in Figures 3 and 4. As it can be seen, the dissolution of montelukast in the tablet of Example 1 is slower with respect to the combination of Rupafin^{®} and Singulair^{®} not achieving 90% after 60 minutes. The dissolution of rupatadine is also slower in the tablet of Example 1 than in the combination of Rupafin^{®} and Singulair^{®}. In view of the dissolution profiles obtained for the tablet of example 1 and for the combination of Rupafin^{®}+Singulair^{®}, we did not expect the formulation of the invention to be bioequivalent to the coadministration of individual montelukast and rupatadine commercial formulations.

## Claims

1. A pharmaceutical composition in the form of a single layer tablet comprising rupatadine or a pharmaceutically acceptable salt thereof and montelukast or a pharmaceutically acceptable salt thereof, wherein:
a) at least one of i) rupatadine or a pharmaceutically acceptable salt thereof or ii) montelukast or a pharmaceutically acceptable salt thereof is present in the form of granules together with at least one pharmaceutically acceptable excipient and
b) the granules do not contain at the same time i) rupatadine or a pharmaceutically acceptable salt thereof and ii) montelukast or a pharmaceutically acceptable salt thereof.

2. The pharmaceutical composition according to claim 1, wherein the above-mentioned granules are prepared by a granulation method different from spray granulation.

3. The pharmaceutical composition according to claim 2, wherein the granulation method employed is selected from the group consisting of dry granulation, wet granulation in a low or high shear mixer and hot melt granulation.

4. The pharmaceutical composition according to claim 3, wherein the granulation method employed is wet granulation in a high-shear mixer.

5. The pharmaceutical composition according to any one of claims 1-4, wherein said pharmaceutical composition comprises at least one excipient selected from the group consisting of binders, diluents, disintegrants, lubricants, glidants, antioxidants, colorants and flavors.

6. The pharmaceutical composition according to any one of claims 1-5, wherein the total amount of diluents is comprised between 20 wt% and 90 wt% w/w, the total amount of binders is comprised between 2 and 14 wt%, the total amount of disintegrants is comprised between 1 and 15 wt % and the total amount of lubricants is comprised between 0.2 and 2.0 wt%, all percentages being expressed relative to the total weight of the pharmaceutical composition.

7. The pharmaceutical composition according to any one of claims 1-6, wherein the amount of rupatadine or a pharmaceutically acceptable salt thereof per pharmaceutical composition in the tablet is of from 0.5 to 22 mg, expressed as weight of rupatadine free base.

8. The pharmaceutical composition according to any one of claims 1-7, wherein the amount of montelukast or a pharmaceutically acceptable salt thereof per pharmaceutical composition in the tablet is of from 1 to 22 mg, expressed as weight of Montelukast free acid.

9. The pharmaceutical composition according to any one of claims 1-8, wherein the weight-to-weight ratio of rupatadine, expressed as weight of rupatadine free base, to the disintegrant is of from 5:1 to 1:5, preferably of from 3.5:1 to 1:3.5, and more preferably of from 2.5:1 to 1:2.5.

10. The pharmaceutical composition according to any one of claims 1-9, wherein the weight-to-weight ratio of montelukast, expressed as weight of montelukast free acid, to the disintegrant is of from 5:1 to 1:5, preferably of from 3.5:1 to 1:3.5, and more preferably of from 2.5:1 to 1:2.5.

11. The pharmaceutical composition according to any one of claims 1-10, wherein rupatadine or a pharmaceutically acceptable salt thereof is present in the form of granules.

12. The pharmaceutical composition according to claim 11, wherein montelukast or a pharmaceutically acceptable salt thereof is present in the form of dry powder.

13. The pharmaceutical composition according to any one of claims 1-12, wherein the pharmaceutically acceptable salt of Rupatadine is Rupatadine fumarate.

14. The pharmaceutical composition according to any one of claims 1-13, wherein the pharmaceutically acceptable salt of Montelukast is Montelukast sodium.

15. A pharmaceutical composition as defined in any one of claims 1-14 for use in medicine.

16. A pharmaceutical composition as defined in any one of claims 1-14 for use in the treatment and/or prevention of asthma accompanied by allergic rhinitis and allergic rhinitis.

## Patentansprüche

1. Eine pharmazeutische Zusammensetzung in Form einer einschichtigen Tablette, umfassend Rupatadin oder ein pharmazeutisch verträgliches Salz davon und Montelukast oder ein pharmazeutisch verträgliches Salz davon, wobei:
a) mindestens eines von i) Rupatadin oder einem pharmazeutisch verträglichen Salz davon oder ii) Montelukast oder einem pharmazeutisch verträglichen Salz davon in Form von Granulat zusammen mit mindestens einem pharmazeutisch verträglichen Hilfsstoff vorliegt und
b) die Granulate nicht gleichzeitig i) Rupatadin oder ein pharmazeutisch verträgliches Salz davon und ii) Montelukast oder ein pharmazeutisch verträgliches Salz davon enthalten.

2. Die pharmazeutische Zusammensetzung nach Anspruch 1, wobei die oben genannten Granulate durch ein anderes Granulationsverfahren als die Sprühgranulation hergestellt werden.

3. Die pharmazeutische Zusammensetzung nach Anspruch 2, wobei das verwendete Granulationsverfahren aus der Gruppe ausgewählt ist, bestehend aus Trockengranulation, Nassgranulation in einem Mischgerät mit niedriger oder hoher Scherung und Heißschmelzgranulation.

4. Die pharmazeutische Zusammensetzung nach Anspruch 3, wobei das verwendete Granulationsverfahren die Nassgranulation in einem Mischgerät mit hoher Scherung ist.

5. Die pharmazeutische Zusammensetzung nach einem der Ansprüche 1-4, wobei die pharmazeutische Zusammensetzung mindestens einen Hilfsstoff umfasst, der ausgewählt ist aus der Gruppe bestehend aus Bindemitteln, Verdünnungsmitteln, Sprengmitteln, Schmiermitteln, Gleitmitteln, Antioxidantien, Farbstoffen und Aromastoffen.

6. Die pharmazeutische Zusammensetzung nach einem der Ansprüche 1-5, wobei die Gesamtmenge an Verdünnungsmitteln zwischen 20 Gew.-% und 90 Gew.-% Gew./Gew. umfasst, die Gesamtmenge an Bindemitteln zwischen 2 und 14 Gew.-% umfasst, die Gesamtmenge an Sprengmitteln zwischen 1 und 15 Gew.-% umfasst und die Gesamtmenge an Schmiermitteln zwischen 0,2 und 2,0 Gew.-% umfasst, wobei alle Prozentsätze bezogen auf das Gesamtgewicht der pharmazeutischen Zusammensetzung angegeben sind.

7. Die pharmazeutische Zusammensetzung nach einem der Ansprüche 1-6, wobei die Menge an Rupatadin oder einem pharmazeutisch verträglichen Salz davon pro pharmazeutischer Zusammensetzung in der Tablette 0,5 bis 22 mg beträgt, angegeben als Gewicht an freier Rupatadinbase.

8. Die pharmazeutische Zusammensetzung nach einem der Ansprüche 1-7, wobei die Menge an Montelukast oder einem pharmazeutisch verträglichen Salz davon pro pharmazeutischer Zusammensetzung in der Tablette 1 bis 22 mg beträgt, angegeben als Gewicht an freier Montelukastsäure.

9. Die pharmazeutische Zusammensetzung nach einem der Ansprüche 1-8, wobei das Gewichtsverhältnis von Rupatadin, angegeben als Gewicht an freier Rupatadinbase, zum Sprengmittel im Bereich von 5:1 bis 1:5, vorzugsweise im Bereich von 3,5:1 bis 1:3,5 und noch bevorzugter im Bereich von 2,5:1 bis 1:2,5 liegt.

10. Die pharmazeutische Zusammensetzung nach einem der Ansprüche 1-9, wobei das Gewichtsverhältnis von Montelukast, angegeben als Gewicht an freier Montelukastsäure, zum Sprengmittel 5:1 bis 1:5, vorzugsweise 3,5:1 bis 1:3,5 und noch bevorzugter 2,5:1 bis 1:2,5 liegt.

11. Die pharmazeutische Zusammensetzung nach einem der Ansprüche 1-10, wobei Rupatadin oder ein pharmazeutisch verträgliches Salz davon in Form von Granulat vorliegt.

12. Die pharmazeutische Zusammensetzung nach Anspruch 11, wobei Montelukast oder ein pharmazeutisch verträgliches Salz davon in Form von Trockenpulver vorliegt.

13. Die pharmazeutische Zusammensetzung nach einem der Ansprüche 1-12, wobei das pharmazeutisch verträgliche Salz von Rupatadin Rupatadinfumarat ist.

14. Die pharmazeutische Zusammensetzung nach einem der Ansprüche 1-13, wobei das pharmazeutisch verträgliche Salz von Montelukast Montelukast-Natrium ist.

15. Eine pharmazeutische Zusammensetzung nach einem der Ansprüche 1-14 zur Verwendung in der Medizin.

16. Eine pharmazeutische Zusammensetzung nach einem der Ansprüche 1-14 zur Verwendung bei der Behandlung und/oder Vorbeugung von Asthma in Verbindung mit allergischer Rhinitis und allergischer Rhinitis.

## Revendications

1. Une composition pharmaceutique sous la forme d'un comprimé monocouche comprenant de la rupatadine ou un sel pharmaceutiquement acceptable de celle-ci et du montélukast ou un sel pharmaceutiquement acceptable de celui-ci, dans laquelle :
a) au moins l'un parmi : i) la rupatadine ou un sel pharmaceutiquement acceptable de celle-ci, ou ii) le montélukast ou un sel pharmaceutiquement acceptable de celui-ci est présent sous forme de granulés en association avec au moins un excipient pharmaceutiquement acceptable, et
b) les granulés ne contiennent pas simultanément i) de la rupatadine ou un sel pharmaceutiquement acceptable de celle-ci et ii) du montélukast ou un sel pharmaceutiquement acceptable de celui-ci.

2. La composition pharmaceutique selon la revendication 1, dans laquelle les granulés susmentionnés sont préparés selon un procédé de granulation différent de la granulation par pulvérisation.

3. La composition pharmaceutique selon la revendication 2, dans laquelle le procédé de granulation utilisé est choisi parmi le groupe constitué par la granulation à sec, la granulation par voie humide dans un mélangeur à faible ou à haut cisaillement et la granulation par fusion à chaud.

4. La composition pharmaceutique selon la revendication 3, dans laquelle le procédé de granulation utilisé est la granulation par voie humide dans un mélangeur à haut cisaillement.

5. La composition pharmaceutique selon l'une quelconque des revendications 1-4, dans laquelle ladite composition pharmaceutique comprend au moins un excipient choisi parmi le groupe constitué des liants, des diluants, des désintégrants, des lubrifiants, des agents de fluidité, des antioxydants, des colorants et des arômes.

6. La composition pharmaceutique selon l'une quelconque des revendications 1-5, dans laquelle la quantité totale de diluants est comprise entre 20 % et 90 % en poids p/p, la quantité totale de liants est comprise entre 2 et 14 % en poids, la quantité totale de désintégrants est comprise entre 1 et 15 % en poids et la quantité totale de lubrifiants est comprise entre 0,2 et 2,0 % en poids, tous ces pourcentages étant exprimés par rapport au poids total de la composition pharmaceutique.

7. La composition pharmaceutique selon l'une quelconque des revendications 1-6, dans laquelle la quantité de rupatadine ou d'un sel pharmaceutiquement acceptable de celle-ci par composition pharmaceutique contenue dans le comprimé est comprise entre 0,5 et 22 mg, exprimée en poids de rupatadine sous forme de base libre.

8. La composition pharmaceutique selon l'une quelconque des revendications 1-7, dans laquelle la quantité de montélukast ou d'un sel pharmaceutiquement acceptable de celui-ci par composition pharmaceutique contenue dans le comprimé est comprise entre 1 et 22 mg, exprimée en poids de montélukast sous forme d'acide libre.

9. La composition pharmaceutique selon l'une quelconque des revendications 1-8, dans laquelle le rapport pondéral de la rupatadine, exprimé en poids de rupatadine sous forme de base libre, au désintégrant est compris entre 5:1 et 1:5, de préférence entre 3,5:1 et 1:3,5, et de préférence encore compris entre 2,5:1 et 1:2,5.

10. La composition pharmaceutique selon l'une quelconque des revendications 1-9, dans laquelle le rapport pondéral du montélukast, exprimé en poids de montélukast sous forme d'acide libre, au désintégrant est compris entre 5:1 et 1:5, de préférence entre 3,5:1 et 1:3,5, et de préférence encore compris entre 2,5:1 et 1:2,5.

11. La composition pharmaceutique selon l'une quelconque des revendications 1-10, dans laquelle la rupatadine ou un sel pharmaceutiquement acceptable de celle-ci est présent sous forme de granulés.

12. La composition pharmaceutique selon la revendication 11, dans laquelle le montélukast ou un sel pharmaceutiquement acceptable de celui-ci est présent sous forme de poudre sèche.

13. La composition pharmaceutique selon l'une quelconque des revendications 1-12, dans laquelle le sel pharmaceutiquement acceptable de la rupatadine est le fumarate de rupatadine.

14. La composition pharmaceutique selon l'une quelconque des revendications 1-13, dans laquelle le sel pharmaceutiquement acceptable du montélukast est le montélukast sodique.

15. Une composition pharmaceutique telle que définie dans l'une quelconque des revendications 1-14, pour un usage médical.

16. Une composition pharmaceutique telle que définie dans l'une quelconque des revendications 1-14, pour utilisation dans le traitement et/ou la prévention de l'asthme associé à une rhinite allergique et de la rhinite allergique.
